# FASCICULE DE BREVET EUROPEEN

(11) **EP 1 118 610 B1**
(45) Date de publication et mention de la délivrance du brevet: **21.08.2002**
(21) Numéro de dépôt: 01400133.3
(22) Date de dépôt: 18.01.2001
(51) Int. Cl.: C07D 211/32, C07D 211/22, C07D 413/04, C07D 417/04, C07D 211/18, C07D 207/08, C07D 295/18, C07D 295/08, A61K 31/445, A61K 31/4965, A61P 7/02

(54) **Nouveaux dérivés de benzènesulfonamide, leur procédé de préparation et les compositions pharmaceutiques qui les contiennent**
Benzolsulfonamidderivate und Verfahren zu ihrer Herstellung und diese enthaltende pharmazeutische Zusammensetzungen
Benzenesulfonamide derivatives, process for their preparation and pharmaceutical compositions containing them

(30) Priorité: 19.01.2000 FR 0000623
(43) Date de publication de la demande: 25.07.2001
(73) Titulaire: LES LABORATOIRES SERVIER, 92200 Neuilly sur Seine (FR)
(72) Inventeur: Lavielle, Gilbert, 78170 La Celle Saint Cloud (FR); Cimetiere, Bernard, 75020 Paris (FR); Verbeuren, Tony, 78540 Vernouillet (FR); Simonet, Serge, 78700 Conflans Sainte Honorine (FR); Descombes, Jean-Jacques, 92500 Rueil Malmaison (FR)

(56) Documents cités:
- EP-A- 0 648 741
- EP-A- 0 864 561
- CIMETIERE B ET AL: "Synthesis and biological evaluation of new tetrahydronaphthalene derivatives as thromboxane receptor antagonists" BIOORGANIC & MEDICINAL CHEMISTRY LETTERS,GB,OXFORD, vol. 8, no. 11, 2 juin 1998 (1998-06-02), pages 1375-1380, XP004137207 ISSN: 0960-894X

## Description

La présente invention concerne des nouveaux dérivés de benzènesulfonamide, leur procédé de préparation et les compositions pharmaceutiques qui les contiennent.

Des composés possèdant un enchaînement benzènesulfonamide ont été décrits dans la demande EP 864561 pour leur caractère donneur de NO et antagonistes des récepteurs au thromboxane A₂ (TXA₂), ainsi que dans la demande EP 648741 pour leurs seules propriétés antagonistes des récepteurs du TXA₂.

Les composés de la présente invention possèdent une structure originale qui leur confère un caractère antagoniste des récepteurs du TXA₂ et antagoniste des récepteurs sérotoninergiques 5HT₂.

L'agrégation plaquettaire et les vasospasmes jouent un rôle essentiel dans l'éthiologie et le développement des maladies cardiovasculaires athéro-thrombotiques. Le TXA₂, métabolisme de l'acide arachidonique, et la sérotonine (5HT), neurotransmetteur, sont tous deux de puissants agents vasoconstricteurs, et peuvent induire ou renforcer l'activation des plaquettes, conduisant à leur agrégation. Les actions vasoconstrictrices et proagrégantes du TXA₂ se font par l'intermédiaire de récepteurs membranaires appelé TP-récepteurs (Medicinal Research Reviews, 1991, 11, 5, p.503) alors que celles de la sérotonine se font par l'intermédiaire des récepteurs 5HT₁ ou 5HT₂ (T.I.P.S., 1991, 121, p.223). Les stratégies de recherche mises en oeuvre afin de trouver des agents qui bloquent la production et/ou l'activation du TXA₂ ont conduit au développement d'antagonistes sélectifs des récepteurs TP, d'inhibiteurs de TXA₂-synthase, ou d'agents mixtes possédant les deux propriétés (Medicinal Research Reviews, ibd., T.I.P.S., 1991, 121, 158). La sérotonine agit, tout comme le TXA₂, en stimulant les plaquettes et les constractions vasculaires, et son activité se trouve renforcée dans les maladies athéro-thrombotiques.

La conception de composés s'opposant à la fois au processus faisant intervenir le thromboxane et à celui faisant intervenir la sérotonine, est d'une grande utilité pour les cliniciens. De tels produits présentent l'avantage d'offrir une protection plus complète, à la fois contre l'activation des plaquettes et contre les vasospasmes. Ils pourront donc être utiles pour le traitement des pathologies liées à une activité exagérée de TXA₂ et de 5-HT en particulier dans le traitement des maladies cardiovasculaires athéro-thrombotiques telles que l'infarctus du myocarde, l'angine de poitrine, les accidents vasculaires cérébraux, la maladie de Raynaud, ou encore l'asthme, les bronchospasmes, mais aussi la migraine et les maladies veineuses.

La présente invention concerne les composés de formule (I) : 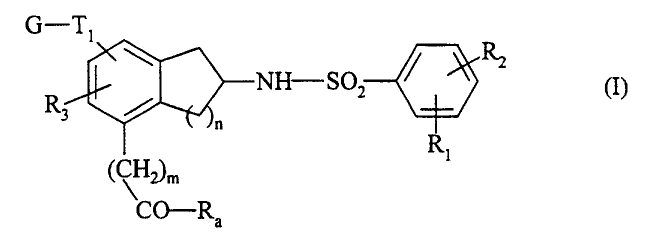 dans laquelle :
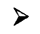 n est un entier compris inclusivement entre 1 et 3,
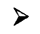 m est un entier compris inclusivement entre 0 et 6,
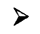 Rₐ représente un groupement hydroxy, alkoxy (C₁-C₆) linéaire ou ramifié, aryloxy, ou arylalkyloxy,
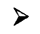 R₁ et R₂ représentent indépendamment un atome d'hydrogène, d'halogène, un groupement alkyle, alkoxy (C₁-C₆) linéaire ou ramifié, hydroxy, ou perhalogénoalkyle (C₁-C₆) linéaire ou ramifié,
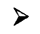 R₃ représente un atome d'hydrogène, ou un groupement alkyle, arylalkyle, cycloalkylalkyle, aryle, ou cycloalkyle,
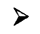 T₁ représente un groupement alkylène, O-alkylène, alkylène-O-, ou (C₁-C₃)alkylène-O-(C₁-C₃)alkylène,
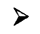 G représente un groupement G₁- ou G₁-T₂-A-, dans lesquels :
   * A représente un groupement aryle,
   * T₂ représente une liaison ou un groupement alkylène, -O-alkylène, alkylène-O-, ou (C₁-C₃)-alkylène-O-(C₁-C₃)alkylène,
   * G₁ représente un groupement -NR₄R₅, dans lequel R₄ et R₅ représentent indépendamment un atome d'hydrogène, un groupement alkyle, cycloalkyle, aryle éventuellement substitué, arylalkyle éventuellement substitué, cycloalkylalkyle, hétéroaryle éventuellement substitué, ou hétéroarylalkyle éventuellement substitué, ou bien G₁ représente un groupement hétérocycloalkyle de formule 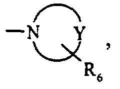 de 5 à 7 chaînons dans lequel Y représente un atome d'azote, d'oxygène ou un groupement CH ou CH₂ et R₆ représente un atome d'hydrogène, un groupement alkyle, cycloalkyle, cycloalkylalkyle, aryle éventuellement substitué, aryalkyle éventuellement substitué, arylcarbonyle éventuellement substitué, arylcarbonylalkyle éventuellement substitué, diarylalkyle éventuellement substitué, diarylalkényle éventuellement substitué, (aryl)(hydroxy)alkyle éventuellement substitué, hétéroaryle éventuellement substitué, hétéroarylalkyle éventuellement substitué, hétéroarylcarbonyle éventuellement substitué, ou hétéroarylcarbonylalkyle éventuellement substitué,
leurs énantiomères, diastéréoisomères ainsi que leurs sels d'addition à un acide ou à une base pharmaceutiquement acceptable,
étant entendu que :
- le terme alkyle désigne une chaîne de 1 à 6 atomes de carbone, linéaire ou ramifié,
- le terme alkényle désigne une chaîne de 2 à 6 atomes de carbone contenant de 1 à 3 doubles liaisons,
- le terme alkylène désigne un groupement bivalent linéaire ou ramifié contenant de 1 à 6 atomes de carbone, sauf précision contraire,
- le terme cycloalkyle désigne un groupement cyclique saturé contenant de 3 à 8 atomes de carbone,
- le terme aryle désigne un groupement phényle ou naphtyle,
- le terme hétéroaryle désigne un groupement mono ou bicyclique de 4 à 11 chaînons, insaturé ou partiellement saturé, et contenant de 1 à 5 hétéroatomes choisis parmi azote, oxygène et soufre,
- les termes diarylalkyle et diarylalkényle désignent respectivement des groupements alkyle et alkényle tels que définis précédemment, substitués par deux groupements aryles, identiques ou différents, tels que définis précédemment,
- le terme « substitué » associé aux expressions aryle, arylalkyle, arylcarbonyle, arylcarbonylalkyle, diarylalkyle, diarylalkényle, hétéroaryle, hétéroarylalkyle, hétéroarylcarbonyle et hétéroarylcarbonylalkyle signifie que les groupements concernés sont substitués sur la partie aromatique par un ou plusieurs atomes d'halogène, groupement alkyle, alkoxy (C₁-C₆) linéaire ou ramifié, hydroxy, cyano, nitro, ou amino (éventuellement substitué par un ou deux groupements alkyle), étant entendu que les groupements hétéroaryle et hétéroarylalkyle peuvent être en plus substitués par un groupement oxo.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthanesulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

Des composés préférés de l'invention sont ceux pour lesquels n vaut 2.

D'autres composés préférés de l'invention sont ceux pour lesquels m vaut 2.

Un aspect avantageux de l'invention concerne les composés de formule (I) pour lesquels R₃ représente un atome d'hydrogène.

Un autre aspect avantageux de l'invention concerne les composés de formule (I) pour lesquels Rₐ représente un groupement hydroxy.

Dans les composés de formule (I), G₁ représente préférentiellement un groupement hétérocycloalkyle de formule 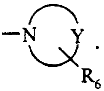 On peut citer par exemple, de façon non limitative les groupements pipéridine, pyrrole, pipérazine ...

De façon avantageuse dans les groupements G₁, R₆ représente un groupement choisi parmi alkyle (par exemple méthyle), arylcarbonyle (par exemple benzoyle), arylcarbonylalkyle (par exemple benzoylméthyle), diarylalkényle (par exemple bisphénylméthylène), (aryl)(hydroxy)alkyle (par exemple (phényl)(hydroxy)méthyle), aryle (par exemple phényle), ou hétéroaryle, tous ces groupements étant éventuellement substitués sur leur partie aromatique lorsqu'ils en possèdent une. On choisira avantageusement comme substituant un atome d'halogène ou un groupement alkoxy.

Parmi les groupements hétéroaryle préférés on peut citer plus particulièrement les groupements 1,2-benzisoxazole, 1,2-benzisothiazole, ...

Un aspect particulièrement avantageux de l'invention concerne les composés de formule (I) pour lesquels n et m valent chacun 2, Rₐ représente un groupement hydroxy, R₂ et R₃ représentent chacun un atome d'hydrogène, R₁ représente un atome d'halogène, et G₁ représente un groupement hétérocycloalkyle de formule 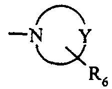 dans laquelle Y représente un atome d'azote ou un groupement -CH ou CH₂, et R₆ est choisi parmi les groupements alkyle, arylcarbonyle, arylcarbonylalkyle, diarylalkényle, (aryl)(hydroxy)alkyle, aryle, et hétéroaryle.

Parmi les composés préférés de l'invention on peut citer plus particulièrement l'acide 3-(6-{[(4-chlorophényl)sulfonyl]amino}-3-{2-[4-(6-fluoro-1,2-benzisothiazol-3-yl)-1-pipéridinyl]éthyl}-5,6,7,8-tétrahydro-1-naphtalényl)propanoïque, et l'acide 3-(6-{[(4-chlorophényl)sulfonyl]amino}-3-{[2-(4-méthyl-1-pipérazinyl)phénoxy]méthyl}-5,6,7,8-tétrahydro-1-naphtalényl)propanoïque.

La présente invention concerne également le procédé de préparation des composés de formule (I) caractérisé en ce que l'on utilise comme produit de départ un composé de formule (II) : 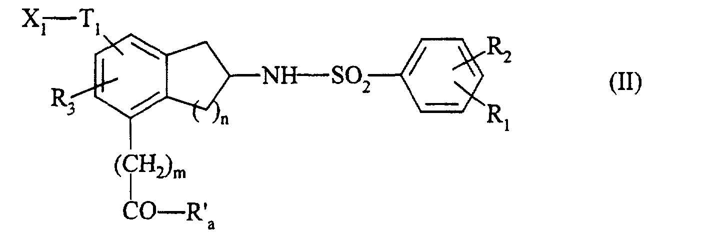 dans laquelle n, m, R₁, R₂, R₃ et T₁ sont tels que définis dans la formule (I), R'ₐ représente un groupement (C₁-C₆) alkoxy linéaire ou ramifié, et X₁ représente un groupe partant (par exemple un atome d'halogène ou un groupement tosyle),
- qui, lorsque dans les composés de formule (I) que l'on souhaite obtenir G représente un groupement G₁ tel que défini dans la formule (I), est traité en milieu basique par un composé de formule G₁H, pour conduire au composé de formule (I/a) : 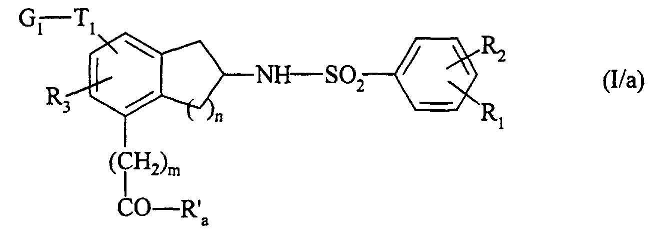 cas particulier des composés de formule (I) pour lequel m, n, R'ₐ, R₁, R₂, R₃, T₁ et G₁ sont tels que définis dans la formule (I),
- ou bien, lorsque dans les composés de formule (I) que l'on souhaite obtenir G représente un groupement G₁-T₂-A- tel que défini dans la formule (I), qui est traité en milieu basique par un composé de formule HO-T₂-A-G_{R}, dans laquelle T₂ et A sont tels que définis dans la formule (I) et G_{R} représente un groupement réactif choisi de façon à ce qu'il puisse réaliser la substitution nucléophile du groupe partant X₁ présent sur le substrat, pour conduire à un composé de formule (IV) : 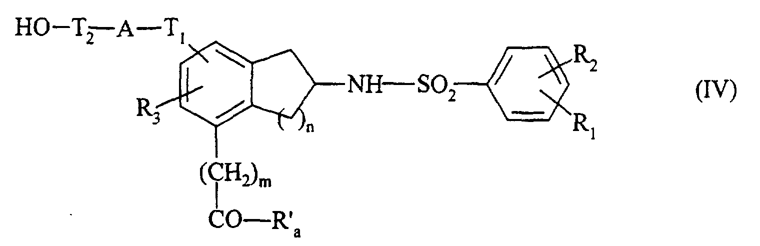 dans laquelle m, n, R'ₐ, R₁, R₂, R₃, T₁, A et T₂ sont tels que définis précédemment,
   dont on transforme le groupement hydroxyle en groupe partant ou en atome d'halogène pour conduire au composé de formule (V) : 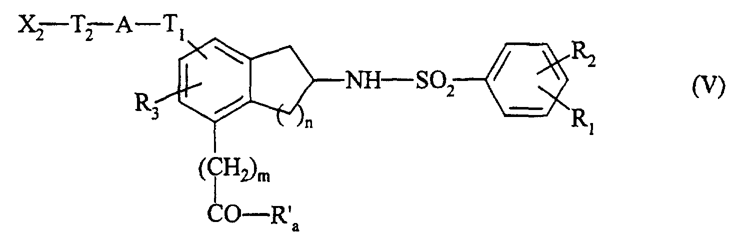 dans laquelle m, n, R'ₐ, R₁, R₂, R₃, T₁, A et T₂ sont tels que définis précédemment, et X₂ représente un groupe partant (par exemple un atome d'halogène ou un groupement tosyle),
   composé de formule (V) qui est traité en milieu basique par un composé de formule G₁H, G₁ étant tel que défini dans la formule (I), pour conduire à un composé de formule (I/b) : 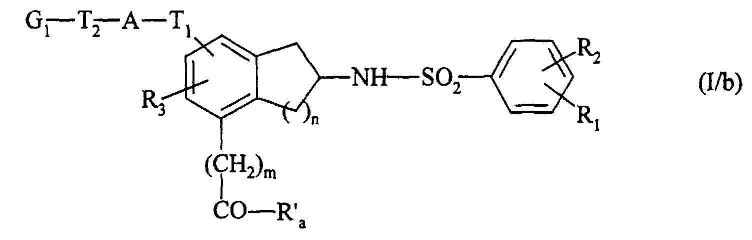 cas particulier des composés de formule (I) pour lequel m, n, R'ₐ, R₁, R₂, R₃, T₁, T₂, A, et G₁ sont tels que définis précédemment,
   composés de formule (I/a) et (I/b), qui peuvent être soumis à une hydrolyse de la fonction ester, en milieu basique ou acide selon les groupements réactifs présents sur la molécule, pour conduire au composé de formule (I/c) : 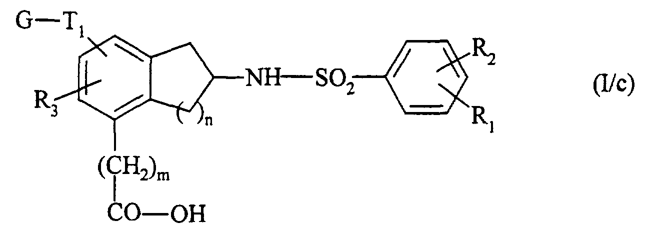 cas particulier des composés de formule (I) pour lequel m, n, R₁, R₂, R₃ et T₁ sont tels que définis précédemment, et G est tel que défini dans la formule (I),
   composés (Ia), (I/b) et (I/c) formant la totalité des composés de formule (I), et :
   - qui peuvent être, le cas échéant, purifiés selon une technique classique de purification,
   - dont on sépare, le cas échéant, les stéréoisomères selon une technique classique de séparation,
   - que l'on transforme, si on le souhaite, en leurs sels d'addition à un acide ou à une base pharmaceutiquement acceptable,
étant entendu qu'à tout moment jugé opportun au cours du procédé précédemment décrit, la fonction ester carboxylique -CO-R'ₐ peut être hydrolysée en acide correspondant, ce dernier pouvant être à nouveau transformé en un autre ester pour les besoins de la synthèse.

La présente invention a également pour objet les compositions pharmaceutiques renfermant comme principe actif un composé de formule (I) seul ou en combinaison avec un ou plusieurs excipients ou véhicules inertes non toxiques, pharmaceutiquement acceptables.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les gélules, les tablettes, les suppositoires, les crèmes, pommades, gels dermiques, etc...

La posologie utile varie selon l'âge et le poids du patient, la nature et la sévérité de l'affection ainsi que la voie d'administration. Celle-ci peut être orale, nasale, rectale ou parentérale. D'une manière générale, la posologie unitaire s'échelonne entre 0,1 et 500 mg pour un traitement en 1 à 3 prises par 24 heures.

Les exemples suivants illustrent l'invention et ne la limitent en aucune façon.

Les produits de départ utilisés sont des produits connus ou préparés selon des modes opératoires connus.

### Préparation A : 3-(3-(Bromométhyl)-6-{[(4-chlorophényl)sulfonyl]amino}-5,6,7,8-tétrahydro-1-naphtalényl)propanoate de méthyle

### stade a : 3-(6-{[(4-Chlorophényl)sulfonyl]amino}-3-formyl-5,6,7,8-tétrahydro-1-naphtalényl)propanoate de méthyle

A une solution de 10 g (23 mmol) de 3-(6-{[(4-chlorophényl)sulfonyl]amino}-3-vinyl-5,6,7,8-tétrahydro-1-naphtalényl)propanoate de méthyle décrit dans la demande EP 864561 dans un mélange de 100 ml de dioxane et 50 ml d'eau, sont ajoutés à température ambiante 2,5 g d'une solution de tétroxyde d'osmium (2,5 % en poids) dans le 2-méthyl-2-propanol, puis 20 g de périodate de sodium. Après agitation une nuit à température ambiante la solution est filtrée, et le filtrat concentré. Le résidu obtenu est repris dans le dichlorométhane lavé à l'eau, et la phase organique est séchée et concentrée, puis purifiée par chromatographie sur gel de silice en utilisant comme éluant un mélange cyclohexane/acétate d'éthyle (60/40) pour conduire au composé attendu.

### stade b : 3 -(6-{[(4-Chlorophényl)sulfonyl]amino}-3-hydroxyméthyl-5,6,7,8-tétrahydro-1-naphtalényl)propanoate de méthyle

A une solution de 4 g (9,2 mmol) du produit décrit au stade précédent dans 100 ml de méthanol est ajouté 1 g (2,6 mmol) de borohydrure de sodium. Le mélange réactionnel est agité 30 minutes à température ambiante. Après ajout d'une solution aqueuse saturée de bicarbonate de sodium, évaporation de la majeure partie du méthanol, le mélange réactionnel est extrait au dichlorométhane. La phase organique est séchée et concentrée. Une purification par chromatographie sur gel de silice, en utilisant comme éluant un mélange acétate d'éthyle/cyclohexane (50/50) conduit au produit attendu.

### stade c : 3-(3-(Bromométhyl)-6-{[(4-chlorophényl)sulfonyl]amino}-5,6,7,8-tétrahydro-1-naphtalényl)propanoate de méthyle

A une solution de 3,10 g (7,1 mmol) du produit décrit au stade précédent dans 50 ml de dichlorométhane, sont ajoutés à température ambiante 2,23 g (8,5 mmol) de triphénylphosphine, puis lentement une solution de 2,83 g (8,5 mmol) de tétrabromocarbone dans 25 ml de dichlorométhane. Après agitation à température ambiante une heure, le solvant est évaporé. Une purification par chromatographie sur gel de silice en utilisant comme éluant un mélange cyclohexane/acétate d'éthyle (80/20) conduit au produit attendu.

### Préparation B : 3-(3-(3-Bromopropyl)-6-{[(4-chlorophényl)sulfonyl]amino}-5,6,7,8-tétrahydro-1-naphtalényl)propanoate de méthyle

### stade a : 3-(7-{[(4-Chlorophényl)sulfonyl]amino}-4-[2-(méthoxycarbonyl)éthyl]-5,6,7,8-tétrahydro-1-naphtalényl)-2-propènoate de tertbutyle

A une solution de 10 g (20,5 mmol) de 3-(3-bromo-6-{[(4-chlorophényl)sulfonyl]amino}-5,6,7,8-tétrahydro-1-naphtalényl)propanoate de méthyle décrit dans la demande EP 864561 dans 250 ml de DMF sont ajoutés 1,25 g (4 mmol) de tri-o-tolylphosphine, 8,5 ml de triéthylamine, 230 mg (1 mmol) d'acétate de palladium, 9 ml d'acrylate de terbutyle. Le mélange réactionnel est agité à 110°C pendant 8 heures. Le solvant est alors évaporé, et une purification par chromatographie sur gel de silice en utilisant comme éluant un mélange cyclohexane/acétate d'éthyle (80/20) conduit au produit attendu.

### stade b : 3-([3-(2-terButoxycarbonyl)éthyl]-6-{[(4-chlorophényl)sulfonyl]amino}-5,6,7,8-tétrahydro-1-naphtalényl)propanoate de méthyle

A une solution de 7,5g (14 mmol) du produit décrit au stade précédent dans 100 ml de méthanol sont ajoutés 0,87 g (3,6 mmol) d'hexahydrate de chlorure de cobalt, puis par portion 1,1 g (2,9 mmol) de borohydrure de sodium. Le mélange réactionnel est agité 2 heures à température ambiante, puis filtré. Le solvant est évaporé, et le résidu est purifié par une chromatographie sur gel de silice en utilisant comme éluant un mélange cyclohexane/acétate d'éthyle (80/20), pour conduire au produit attendu.

### stade c : Acide 3-(7-{[(4-Chlorophényl)sulfonyl]amino}-4-[2 -(méthoxycarbonyl) éthyl]-5,6,7,8-tétrahydro-2-naphtalényl)propanoïque

Une solution de 6,4 g (12 mmol) du produit décrit au stade précédent dans 50 ml d'acide trifluoroacétique est agitée 12 heures à température ambiante. Le solvant est alors évaporé et le résidu est repris dans l'acétate d'éthyle. La phase organique est lavée à la saumure, puis séchée et évaporée. Le produit est obtenu après purification par chromatographie sur gel de silice avec comme éluant un mélange dichlorométhane/méthanol (98/2).

### stade d : 3-(6-{[(4-Chlorophényl)sulfonyl]amino}-3-(3 -hydroxypropyl)-5,6,7,8-tétrahydro-1-naphtalényl)propanoate de méthyle

A une solution de 2,8 g (5,2 mmol) du produit décrit au stade précédent dans 80 ml de THF à température ambiante sont ajoutés lentement 9 ml d'une solution 1M de BH₃/THF dans le THF. Après une nuit d'agitation à température ambiante, 10 ml d'eau sont ajoutés. La majeure partie du solvant est évaporée, et le résidu est repris dans l'acétate d'éthyle. La phase organique est alors lavée à la saumure, séchée et évaporée, pour conduire au produit attendu.

### stade e : 3-(6-{[(4-Chlorophényl)sulfonyl]amino}-3-(3-bromopropyl)-5,6,7,8-tétrahydro-1-naphtalényl)propanoate de méthyle

Le produit est obtenu selon le procédé décrit dans la préparation A, stade c, à partir du composé décrit au stade précédent.

### Préparation C : 3-(6-{[(4-Chlorophényl)sulfonyl]amino}-3-({4-[2-(tosyloxy)éthyl] phénoxy}méthyl)-5,6,7,8-tétrahydro-1-naphtalényl)propanoate de méthyle

### stade a : 3-(6-{[(4-Chlorophényl)sulfonyl]amino}-3-[4-(2-hydroxyéthyl) phénoxyméthyl]-5,6,7,8-tétrahydro-1-naphtalényl)propanoate de méthyle

A une solution de 0,58 g (4,2 mmol) de 2-(4-hydroxyphényl)éthanol dans 100 ml de THF, sont ajoutés 165 mg (4,2 mmol) d'hydrure de sodium (60 % dans l'huile minérale), puis une solution de 1,05 g (2,1 mmol) du produit décrit dans la préparation A dans 50 ml de THF et 1,11 g d'éther couronne C₁₈₋₆. Le mélange réactionnel est porté à reflux une heure. La majeure partie du THF est évaporée, le milieu est hydrolysé, et ramené à pH acide avec de l'acide chlorhydrique 1 N. Après extraction au dichlorométhane, séchage et purification par chromatographie sur gel de silice avec comme éluant un mélange acétate d'éthyle/cyclohexane (50/50), le produit attendu est obtenu.

### stade b : 3-(6-{[(4-Chlorophényl)sulfonyl]amino}-3-({4-[2-(tosyloxy)éthyl] phénoxy}méthyl)-5,6,7,8-tétrahydro-1-naphtalényl)propanoate de méthyle

A une solution de 0,75 g (1,35 mmol) du produit obtenu au stade précédent dans 50 ml de dichlorométhane, sont ajoutés 1 g (5,4 mmol) de chlorure de tosyle puis 0,5 ml de pyridine. Après agitation à température ambiante une nuit, le mélange est lavé à l'acide chlorhydrique 1 N et séché. Après évaporation du solvant et purification par chromatographie sur gel de silice en utilisant comme éluant un mélange cyclohexane/acétate d'éthyle (60/40), on obtient le produit attendu.

### Préparation D : (2,3-Diméthoxy)(4-pipéridinyl)méthanol

### stade a : 1-Benzyl-4-pipéridinylcarboxamide

Un mélange de 20 g (156 mmol) d'isonipecotamide, de 32,4 g (234 mmol) de carbonate de potassium, de 2 g (12 mmol) d'iodure de potassium et de 18,6 ml (156 mmol) de bromure de benzyle dans 400 ml d'acétonitrile est porté à reflux pendant 5 heures. Le solvant est évaporé et le résidu repris dans un mélange dichlorométhane/eau. Après décantation. extraction au dichlorométhane, lavage des phases organiques à la saumure et séchage. l'évaporation du solvant conduit au produit attendu.

### stade b : 1-Benzyl-4-pipéridylcarbonitrile

A un mélange de 83 ml (890 mmol) d'oxychlorure de phosphore et de 17 (290 mmol) de chlorure de sodium sont ajoutés par portion 26 g (119 mmol) du produit décrit au stade précédent. Le mélange est chauffé à reflux une heure. Après refroidissement, le mélange réactionnel est versé sur 75 ml d'ammoniaque concentrée. Après extraction au dichlorométhane, lavage de la phase organique à l'eau, et séchage, l'évaporation du solvant conduit au produit attendu.

### stade c : 1-Benzyl-4-pipéridylcarbaldéhyde

A une solution de 22 g (110 mmol) du produit décrit au stade précédent dans 500 ml de THF sont ajoutés, à 0°C, 120 ml d'une solution 1M de diisobutylaluminium hydride dans l'hexane. Le mélange est agité à température ambiante 2 heures. Après hydrolyse avec une solution d'acide chlorhydrique 10 %, le mélange est neutralisé avec une solution aqueuse de soude concentrée. Après extraction à l'éther diéthylique, séchage, évaporation du solvant, une purification par chromatographie sur gel de silice en utilisant comme éluant un mélange cyclohexane/acétate d'éthyle (50/50) conduit au produit attendu.

### stade d : (1-Benzyl-4-pipéridyl)(2,3-diméthoxyphényl)méthanol

A une solution de 7,07 g (51 mmol) de veratrole dans 150 ml de THF à 0°C sont ajoutés 32,5 ml d'une solution 1,6 M de n-butyllithium dans l'hexane. Après agitation 2 heures à 0°C, le mélange réactionnel est refroidi à -78°C, et une solution de 8,6 g (42 mmol) du produit décrit au stade précédent dans 200 ml de THF est ajoutée. L'agitation est poursuivie une heure à -78°C. Après retour à température ambiante, le milieu est hydrolysé à l'eau, extrait à l'acétate d'éthyle, séché et concentré. Une purification par chromatographie sur gel de silice en utilisant comme éluant de l'acétate d'éthyle conduit au produit attendu.

### stade e : (2,3-Diméthoxyphényl)(4-pipéridyl)méthanol

Un mélange de 7,5 g (22 mmol) du produit décrit au stade précédent, 1,5 g de palladium sur charbon (10 %) et 5,5 g (87 mmol) de formiate d'ammonium dans 150 ml de méthanol et 30 ml d'eau est porté à reflux une heure. Après retour à température ambiante et filtration, le solvant est évaporé. Le résidu est repris dans le dichlorométhane, et traité à la soude 2 N jusqu'à pH = 10. Après extraction au dichlorométhane, séchage et évaporation du solvant est obtenu le produit attendu.

### Préparation E : 2-(4-Méthyl-1-pipérazinyl)phénol

### stade a : 4-(2-Hydroxyphényl)-1-pipérazinylcarboxylate d'éthyle

A une solution de 18 g (100 mmol) de 2-(1-pipérazinyl)phénol dans 250 ml de dichlorométhane sont ajoutés 15 ml (156 mmol) de chloroformiate d'éthyle. Après agitation à température ambiante pendant une heure, le milieu est hydrolysé, puis extrait au dichlorométhane. La phase organique est lavée par une solution d'acide chlorhydrique 1 N et séchée. Après concentration le résidu obtenu est recristallisé dans l'éther pour conduire au produit attendu.

### stade b : 4-[2-(Tosyloxy)phényl]-1-pipérazinylcarboxylate d'éthyle

A une solution de 23 g (91 mmol) du produit décrit au stade précédent, dans 100 ml de dichlorométhane, sont ajoutés à température ambiante 25 g (130 mmol) de chlorure de paratoluènesulfonyle et 20 ml de triéthylamine. Après agitation 72 heures à température ambiante, le solvant est évaporé. Une chromatographie sur gel de silice en utilisant comme éluant un mélange acétate d'éthyle/cyclohexane (30/70) conduit au produit attendu.

### stade c : [2-(4-Méthyl-1-pipérazinyl)phénol]-4-toluène sulfonate

A une solution de 23,2 g (57 mmol) du produit décrit au stade précédent dans 100 ml de THF sont ajoutés à 0°C 3 g (79 mmol) d'hydrure double d'aluminium et de lithium. Le mélange est agité 2 heures à température ambiante puis hydrolysé. Après concentration et extraction au dichlorométhane, la phase organique est séchée et concentrée pour conduire au composé attendu.

### stade d : 2-(4-Méthyl-1-pipérazinyl)phénol

Un mélange de 18 g (52 mmol) du produit décrit au stade précédent et de 44 g (785 mmol) de potasse dans 400 ml d'éthanol est porté à reflux pendant 2 heures. Après retour à température ambiante, le pH est ajusté à 7 par l'acide chlorhydrique 1 N. Après concentration, le milieu est extrait au dichlorométhane, et la phase organique est séchée puis concentrée pour conduire au produit attendu.

### Préparation F : 4-(4-méthyl-1-pipérazinyl)-phénol

Le produit est obtenu selon le procédé décrit dans la préaparation E, en remplaçant dans le stade a, le 2-(1-pipérazinyl)phénol par le 4-(1-pipérazinyl) phénol

### EXEMPLE 1 : Acide 3-(6-{[(4-chlorophéuyl)sulfonyl]amino}-3-{2-[4-(4-fluorobenzoyl) -1-pipéridinyl]éthyl}-5,6,7,8-tétrahydro-1-naphtalényl)propanoïque

### Stade a : 3-(6-{[(4-Chlorophényl)sulfonyl]amino}-3-{2-[4-(4-fluorobenzoyl)-1-pipéridinyl]éthyl}-5,6,7,8-tétrahydro-1-naphtalényl)propanoate de méthyle

A une solution de 3,5 g (5,7 mmol) de 3-[6-{[(4-chlorophényl)sulfonyl]amino}-3-(2-{[(4-méthylphényl)sulfonyl]oxy}éthyl)-5,6,7,8-tétrahydro-1-naphtalényl]propanoate de méthyle décrit dans la demande EP 864561, dans 100 ml de DMF, sont ajoutés 6,5 g (17,3 mmol) de tosylate de 4-(4-fluorobenzoyl)pipéridine, et 2,4 g (17,3 mmol) de carbonate de potassium. Le mélange réactionnel est porté à reflux pendant une heure, puis concentré. Le résidu est repris dans le dichlorométhane et lavé à l'eau. La phase organique est séchée et concentrée puis purifiée par chromatographie sur gel de silice, en utilisant comme éluant un mélange dichlorométhane/méthanol/ammoniaque (98/2/0,2), pour conduire au composé attendu.

### Stade b : Acide 3-(6-{[(4-chlorophényl)sulfonyl]amino}-3-{2-[4-(4-fluorobenzoyl)-1-pipéridinyl]éthyl}-5,6,7,8-tétrahydro-1-naphtalényl)propanoïque

Une solution de 2,2 g (3,5 mmol) du produit décrit au stade précédent est portée à reflux pendant deux heures, en présence de 3,5 ml de soude 2 N. Le milieu réactionnel est filtré et le filtrat concentré. 100 ml d'eau sont ajoutés et le pH est amené à 5 à l'aide d'acide acétique. Le précipité formé est alors filtré et recristallisé dans le dichlorométhane pour conduire au composé du titre.
Point de fusion : 210 °C

### Microanalyse élémentaire :

| | C% | H% | N% | S% |
|---|---|---|---|---|
| Calculé | 63,20 | 5,79 | 4,47 | 5,11 |
| Trouvé | 62,89 | 5,87 | 4,46 | 4,82 |

### EXEMPLE 2 : Acide 3-[6-{[(4-chlorophényl)sulfonyl]amino}-3-(2-{4-[(2,3-diméthoxyphényl)(hydroxy)méthyl]-1-pipéridinyl}éthyl)-5,6,7,8-tétrahydro-1-naphtalényl]propanoïque

Le produit attendu est obtenu selon le procédé décrit dans l'exemple 1, en remplaçant au stade a le tosylate de 4-(4-fluorobenzoyl)pipéridine par le composé décrit dans la préparation D.

### Microanalyse élémentaire :

| | C% | H% | N% | S% |
|---|---|---|---|---|
| Calculé | 62,63 | 6,46 | 4,17 | 4,78 |
| Trouvé | 62,13 | 7,00 | 4,17 | 4,64 |

### EXEMPLE 3 : Acide 3-(6-{[(4-chlorophényl)sulfonyl]amino}-3-{2-[4-(6-fluoro-1,2-benzisoxazol-3-yl)-1-pipéridinyl]éthyl}-5,6,7,8-tétrahydro-1-naphtalényl)propanoïque

Le produit attendu est obtenu selon le procédé décrit dans l'exemple 1, en remplaçant au stade a le tosylate de 4-(4-fluorobenzoyl)pipéridine par le chlorhydrate de 6-fluoro-3-pipéridin-4-yl-benzo[d]isoxazole.
Point de fusion : 125 °C

### Microanalyse élémentaire :

| | C% | H% | N% | S% |
|---|---|---|---|---|
| Calculé | 61,92 | 5,51 | 6,56 | 5,01 |
| Trouvé | 61,33 | 5,45 | 6,36 | 4,91 |

### EXEMPLE 4 : Acide 3-(6-{[(4-chlorophényl)sulfonyl]amino}-3-{2-[4-(6-fluoro-1,2-benzisothiazol-3-yl)-1-pipéridinyl]éthyl}-5,6,7,8-tétrahydro-1-naphtalényl)propanoïque

Le produit attendu est obtenu selon le procédé décrit dans l'exemple 1, en remplaçant au stade a le tosylate de 4-(4-fluorobenzoyl)pipéridine par le chlorhydrate de 6-fluoro-3-pipéridin-4-yl-benso[d]isothiazole.
Point de fusion : 232 °C

### Microanalyse élémentaire :

| | C% | H% | N% | S% |
|---|---|---|---|---|
| Calculé | 60,40 | 5,38 | 6,40 | 9,77 |
| Trouvé | 60,17 | 5,36 | 6,39 | 9,50 |

### EXEMPLE 5 : Acide 3-(6-{[(4-chlorophényl)sulfonyl]amino}-3-{2-[4-(1,2-benzisothiazol-3-yl)-1-pipérazinyl]éthyl}-5,6,7,8-tétrahydro-1-naphtalényl)propanoïque

Le produit attendu est obtenu selon le procédé décrit dans l'exemple 1, en remplaçant au stade a le tosylate de 4-(4-fluorobenzoyl)pipéridine par le chlorhydrate de 3-pipérazin-1-yl-benso[d]isothiazole.

### EXEMPLE 6 : Acide 3-(3-(2-{4-[bis(4-fluorophényl)méthylène]-1-pipéridinyl}éthyl)-6-{[(4-chlorophényl)sulfonyl]amino}-5,6,7,8-tétrahydro-1-naphtalényl)propanoïque

Le produit attendu est obtenu selon le procédé décrit dans l'exemple 1, en remplaçant au stade a le tosylate de 4-(4-fluorobenzoyl)pipéridine par la bis(4-fluorophényl)méthylènepipéridine.
Point de fusion : 242 °C

### Microanalyse élémentaire :

| | C% | H% | N% | S% |
|---|---|---|---|---|
| Calculé | 66,42 | 5,57 | 3,97 | 4,55 |
| Trouvé | 66,27 | 5,52 | 4,05 | 4,32 |

### EXEMPLE 7 : Acide 3-[6-{[(4-chlorophényl)sulfonyl]amino}-3-(2-{3-[2-(4-fluorophényl)-2-oxoéthyl]-1-pyrrolidinyl}éthyl)-5,6,7,8-tétrahydro-1-naphtalényl)propanoïque

Le produit attendu est obtenu selon le procédé décrit dans l'exemple 1, en remplaçant au stade a le tosylate de 4-(4-fluorobenzoyl)pipéridine par le chlorhydrate de 1-(4-fluorophényl)-2-pyrrolidin-3-yl-éthanone.
Point de fusion : 143 °C

### Microanalyse élémentaire :

| | C% | H% | N% | S% |
|---|---|---|---|---|
| Calculé | 63,20 | 5,79 | 4,47 | 5,11 |
| Trouvé | 63,79 | 5,79 | 4,52 | 4,99 |

### EXEMPLE 8 : Acide 3-(6-{[(4-chlorophényl)sulfonyl]amino}-2-{2-[4-(4-fluorobenzoyl)-1-pipéridinyl]éthyl}-5,6,7,8-tétrahydro-1-naphtalényl)propanoïque

Le produit attendu est obtenu selon le procédé décrit dans l'exemple 1 en remplaçant le 3-[6-{[(4-chlorophényl)sulfonyl]amino}-3-(2-{[(4-méthylphényl)sulfonyl]oxy}éthyl)-5,6,7,8-tétrahydro-1-naphtalényl]propanoate de méthyle par le 3-[6-{[(4-chlorophényl) sulfonyl]amino}-2-(2-{[(4-méthylphényl)sulfonyl]oxy}éthyl)-5,6,7,8-tétrahydro-1-naphtalényl] propanoate de méthyle décrit dans la demande EP 864561.
Point de fusion : 223 °C

### Microanalyse élémentaire :

| | C% | H% | N% | S% |
|---|---|---|---|---|
| Calculé | 63,20 | 5,79 | 4,47 | 5,11 |
| Trouvé | 63,14 | 5,80 | 4,56 | 5,17 |

### EXEMPLE 9 : Acide 3-(6-{[(4-chlorophényl)sulfonyl]amino}-3-{[4-(4-fluorobenzoyl) -1-pipéridinyl]méthyl}-5,6,7,8-tétrahydro-1-naphtalényl) propanoïque

Le produit attendu est obtenu selon le procédé décrit dans l'exemple 1 en remplaçant le 3-[6-{[(4-chlorophényl)sulfonyl]amino}-3-(2-{[(4-méthylphényl)sulfonyl]oxy}éthyl)-5,6,7,8 -tétrahydro-1-naphtalényl]propanoate de méthyle par le produit décrit dans la préparation A.
Point de fusion : 147 °C

### Microanalyse élémentaire :

| | C% | H% | N% | S% |
|---|---|---|---|---|
| Calculé | 62,69 | 5,59 | 4,59 | 5,23 |
| Trouvé | 62,99 | 5,49 | 4,48 | 5,17 |

### EXEMPLE 10 : Acide 3-(6-{[(4-chlorophényl)sulfonyl]amino}-3-{3-[4-(4-fluorobenzoyl)-1-pipéridinyl]propyl}-5,6,7,8-tétrahydro-1-naphtalényl) propanoïque

Le produit attendu est obtenu selon le procédé décrit dans l'exemple 1 en remplaçant le 3-[6-{[(4-chlorophényl)sulfonyl]amino}-3-(2-{[(4-méthylphényl)sulfonyl]oxy}éthyl)-5,6,7,8 -tétrahydro-1-naphtalényl]propanoate de méthyle par le produit décrit dans la préparation B.
Point de fusion : 118 °C

### Microanalyse élémentaire :

| | C% | H% | N% | S% |
|---|---|---|---|---|
| Calculé | 63,69 | 5,97 | 4,37 | 5,00 |
| Trouvé | 63,55 | 6,02 | 4,37 | 4,98 |

### EXEMPLE 11 : Acide 3-(6-{[(4-chlorophényl)sulfonyl]amino}-3-{3-[4-(4-fluorobenzoyl)-1-pipérazinyl]propyl}-5,6,7,8-tétrahydro-1-naphtalényl)propanoïque

Le produit attendu est obtenu selon le procédé décrit dans l'exemple 1 en remplaçant le 3-[6-{[(4-chlorophényl)sulfonyl]amino}-3-(2-{[(4-méthylphényl)sulfonyl]oxy}éthyl)-5,6,7,8 -tétrahydro-1-naphtalényl]propanoate de méthyle par le produit décrit dans la préparation B, et le tosylate de 4-(4-fluorobenzoyl)pipéridine par la (4-fluorophényl)pipérazine

### Microanalyse élémentaire :

| | C % | H % | N % |
|---|---|---|---|
| Calculé | 59,07 | 5,89 | 6,46 |
| Trouvé | 59,10 | 5,83 | 6,34 |

### EXEMPLE 12 : Acide 3-{6-{[(4-chlorophényl)sulfonyl]amino}-3-[(4-{2-[4-(4-fluorobenzoyl)-1-pipéridinyl]éthyl}phénoxy)méthyl]-5,6,7,8-tétrahydro-1-naphtalényl}propanoïque

Le produit attendu est obtenu selon le procédé décrit dans l'exemple 1 en remplaçant le 3-[6-{[(4-chlorophényl)sulfonyl]amino}-3-(2-{[(4-méthylphényl)sulfonyl]oxy}éthyl)-5,6,7,8 -tétrahydro-1-naphtalényl]propanoate de méthyle par le produit décrit dans la préparation C.
Point de fusion : 196 °C

### Microanalyse élémentaire :

| | C% | H% | N% | S% |
|---|---|---|---|---|
| Calculé | 62,52 | 5,77 | 3,82 | 4,37 |
| Trouvé | 64,89 | 6,29 | 3,84 | 4,34 |

### EXEMPLE 13 : Acide 3-(6-{[(4-chlorophényl)sulfonyl]amino}-3-{[2-(4-méthyl-1-pipérazinyl)phénoxy]méthyl}-5,6,7,8-tétrahydro-1-naphtalényl) propanoïque

### Stade a : 3-(6-{[(4-chlorophényl)sulfonyl]amino}-3-{[2-(4-méthyl-1-pipérazinyl) phénoxy]méthyl}-5,6,7,8-tétrahydro-1-naphtalényl)propanoate de méthyle

Un mélange de 1,30 g (2,6 mmol) du produit décrit dans la préparation A, de 0,5 g (2,6 mmol) du produit décrit dans la préparation E, de 200 mg (5,2 mmol) d'hydrure de sodium (60% dans l'huile), et de 670 mg d'éther couronne C₁₈₋₆ est porté à reflux pendant deux heures. Après refroidissement, 2 ml d'acide acétique sont ajoutés et le milieu réactionnel est concentré. Le résidu est repris dans le dichlorométhane, et lavé à l'eau. La phase organique est séchée, concentrée et purifiée par chromatographie sur gel de silice en utilisant comme éluant un mélange dichlorométhane/méthanol/ammoniaque (95/5/0,5) pour conduire au produit attendu.

### Stade b : Acide 3-(6-{[(4-chlorophényl)sulfonyl]amino}-3-{[2-(4-méthyl-1-pipérazinyl)phénoxy]méthyl}-5,6,7,8-tétrahydro-1-naphtalényl) propanoïque

Le produit attendu est obtenu selon le procédé décrit au stade b l'exemple 1 utilisant comme produit de départ le composé décrit au stade précédent.
Point de fusion : 122 °C

### Microanalyse élémentaire :

| | C% | H% | N% | S% |
|---|---|---|---|---|
| Calculé | 62,25 | 6,07 | 7,02 | 5,36 |
| Trouvé | 61,52 | 6,09 | 6,84 | 5,22 |

### EXEMPLE 14 : Acide 3-(6-{[(4-chlorophényl)sulfonyl]amino}-3-{[4-(4-méthyl-1-pipérazinyl) phénoxy] méthyl}-5,6,7,8-tétrahydro-1-naphtalényl) propanoïque

Le produit attendu est obtenu selon le procédé décrit dans l'exemple 13 en remplaçant dans le stade a le produit décrit dans la préparation E par le produit décrit dans la préparation F.
Point de fusion : 148°C

### Microanalyse élémentaire :

| | C% | H% | N% | S% |
|---|---|---|---|---|
| Calculé | 62,25 | 6,07 | 7,02 | 5,36 |
| Trouvé | 61,94 | 6,36 | 6,66 | 5,11 |

### EXEMPLE 15 : Acide 3-(6-{[(4-chlorophényl)sulfonyl]amino}-3-{2-[4-(6-fluoro-1,2-benzo[b] thiophen-3-yl)-1-pipéridinyl]éthyl}-5,6,7,8-tétrahydro-1-naphtalényl)propanoïque

Le produit attendu est obtenu selon le procédé décrit dans l'exemple 1 en remplaçant au stade a le tosylate de 4-(4-fluorobenzoyl)pipéridine par le chlorhydrate de 4-(6-fluorobenzo[b]thiophen-3-yl)-pipéridine.
Point de fusion : 140°C

### Microanalyse élémentaire :

| | C% | H% | N% | S% |
|---|---|---|---|---|
| Calculé | 62,32 | 5,54 | 4,28 | 9,79 |
| Trouvé | 62,01 | 5,36 | 4,28 | 9,80 |

### EXEMPLE 16 : Acide 3-(6-{[(4-chlorophényl)sulfonyl]amino}-3-{2-[4-(6-fluoro-1H-indazol-3-yl)-1-pipéridinyl]éthyl}-5,6,7,8-tétrahydro-1-naphtalényl) propanoïque

Le produit attendu est obtenu selon le procédé décrit dans l'exemple 1, en remplaçant au stade a le tosylate de 4-(4-fluorobenzoyl)pipéridine par le dichlohydrate de 6-fluoro-3-pipéridin-4-yl-1*H*-indazole.
Point de fusion : 142°C

### Microanalyse élémentaire :

| | C% | H% | N% | S% |
|---|---|---|---|---|
| Calculé | 62,01 | 5,68 | 8,77 | 5,02 |
| Trouvé | 61,98 | 5,74 | 8,70 | 4,83 |

### ETUDE PHARMACOLOGIQUE

### EXEMPLE A : Agrégation plaquettaire chez l'homme

Le sang veineux est obtenu de volontaires humains n'ayant pas pris de l'aspirine pendant au moins 14 jours précédent l'expérience. Le sang est prélevé sur citrate de sodium (0.109 M) (1 vol. de citrate sur 9 vol. de sang). Le plasma riche en plaquettes (PRP) est obtenu après centrifugation (20°C) à 200 g pendant 10 minutes. Le nombre de plaquettes est en moyenne de 250000 PL/mm³. Le PRP est conservé à la température de la pièce jusqu'au moment du test et est utilisé dans les 2 heures qui suivent le prélèvement. L'agoniste TXA₂, le U46619 est utilisé à la concentration de 1 µM et la 5-hydroxytryptamine à la concentration de 10 µM, ce dernier en présence d'adénosine diphosphate à 0.3 µM et d'adrénaline à 1 µM.

Les composés de l'invention inhibent l'agrégation plaquettaire induite par l'agoniste TXA₂ ainsi que celle produite par la 5-hydroxytryptamine. A titre d'exemple les IC₅₀ du composé de l'exemple 4 sont de 170 nM et de 230 nM respectivement sur les deux cibles. Les valeurs indiquent que les composés de l'invention sont de puissants anti-agrégants plaquettaires agissant de façon balancée sur les deux voies d'activation, celle du TXA₂ et celle de la sérotonine.

### EXEMPLE B : Composition pharmaceutique

| Formule de préparation pour 1000 comprimés dosés à 5 mg : | |
|---|---|
| Composé de l'exemple 4 | 5 g |
| Hydroxypropylméthylcellulose | 2 g |
| Amidon de blé | 10 g |
| Lactose | 100 g |
| Stéarate de magnésium | 3 g |

## Revendications

1. Composés de formule (I) : 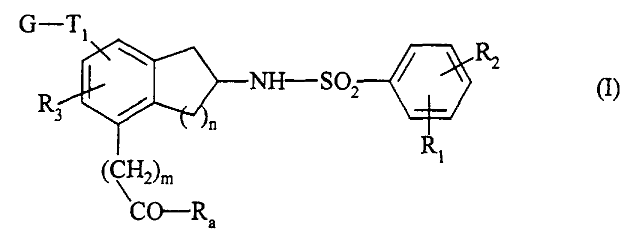 dans laquelle :
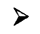 n est un entier compris inclusivement entre 1 et 3,
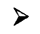 m est un entier compris inclusivement entre 0 et 6,
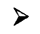 Rₐ représente un groupement hydroxy, alkoxy (C₁-C₆) linéaire ou ramifié, aryloxy, ou arylalkyloxy,
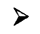 R₁ et R₂ représentent indépendamment un atome d'hydrogène, d'halogène, un groupement alkyle, alkoxy (C₁-C₆) linéaire ou ramifié, hydroxy, ou perhalogénoalkyle (C₁-C₆) linéaire ou ramifié,
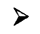 R₃ représente un atome d'hydrogène, ou un groupement alkyle, arylalkyle, cycloalkylalkyle, aryle, ou cycloalkyle,
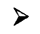 T₁ représente un groupement alkylène, O-alkylène, alkylène-O-, ou (C₁-C₃)alkylène-O-(C₁-C₃)alkylène,
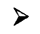 G représente un groupement G₁- ou G₁-T₂-A-, dans lesquels :
* A représente un groupement aryle,
* T₂ représente une liaison ou un groupement alkylène, -O-alkylène, alkylène-O-, ou (C₁-C₃)-alkylène-O-(C₁-C₃)alkylène,
* G₁ représente un groupement -NR₄R₅, dans lequel R₄ et R₅ représentent indépendamment un atome d'hydrogène, un groupement alkyle, cycloalkyle, aryle éventuellement substitué, arylalkyle éventuellement substitué, cycloalkylalkyle, hétéroaryle éventuellement substitué, ou hétéroarylalkyle éventuellement substitué, ou bien G₁ représente un groupement hétérocycloalkyle de formule 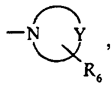 de 5 à 7 chaînons dans lequel Y représente un atome d'azote, d'oxygène ou un groupement CH ou CH₂ et R₆ représente un atome d'hydrogène, un groupement alkyle, cycloalkyle, cycloalkylalkyle, aryle éventuellement substitué, aryalkyle éventuellement substitué, arylcarbonyle éventuellement substitué, arylcarbonylalkyle éventuellement substitué, diarylalkyle éventuellement substitué, diarylalkényle éventuellement substitué, (aryl)(hydroxy)alkyle éventuellement substitué, hétéroaryle éventuellement substitué, hétéroarylalkyle éventuellement substitué, hétéroarylcarbonyle éventuellement substitué, ou hétéroarylcarbonylalkyle éventuellement substitué,
leurs énantiomères, diastéréoisomères ainsi que leurs sels d'addition à un acide ou à une base pharmaceutiquement acceptable,
étant entendu que :
- le terme alkyle désigne une chaîne de 1 à 6 atomes de carbone, linéaire ou ramifié,
- le terme alkényle désigne une chaîne de 2 à 6 atomes de carbone contenant de 1 à 3 doubles liaisons,
- le terme alkylène désigne un groupement bivalent linéaire ou ramifié contenant de 1 à 6 atomes de carbone, sauf précision contraire,
- le terme cycloalkyle désigne un groupement cyclique saturé contenant de 3 à 8 atomes de carbone,
- le terme aryle désigne un groupement phényle ou naphtyle,
- le terme hétéroaryle désigne un groupement mono ou bicyclique de 4 à 11 chaînons, insaturé ou partiellement saturé, et contenant de 1 à 5 hétéroatomes choisis parmi azote, oxygène et soufre,
- les termes diarylalkyle et diarylalkényle désignent respectivement des groupements alkyle et alkényle tels que définis précédemment, substitués par deux groupements aryles, identiques ou différents, tels que définis précédemment,
- le terme « substitué » associé aux expressions aryle, arylalkyle, arylcarbonyle, arylcarbonylalkyle, diarylalkyle, diarylalkényle, hétéroaryle, hétéroarylalkyle, hétéroarylcarbonyle et hétéroarylcarbonylalkyle signifie que les groupements concernés sont substitués sur la partie aromatique par un ou plusieurs atomes d'halogène, groupement alkyle, alkoxy (C₁-C₆) linéaire ou ramifié, hydroxy, cyano, nitro, ou amino (éventuellement substitué par un ou deux groupements alkyle), étant entendu que les groupements hétéroaryle et hétéroarylalkyle peuvent être en plus substitués par un groupement oxo.

2. Composés de formule (I) selon la revendication 1 pour lesquels n vaut 2, leurs énantiomères, diastéréoisomères ainsi que leurs sels d'addition à un acide ou à une base pharmaceutiquement acceptable.

3. Composés de formule (I) selon la revendication 1 pour lesquels m vaut 2, leurs énantiomères, diastéréoisomères ainsi que leurs sels d'addition à un acide ou à une base pharmaceutiquement acceptable.

4. Composés de formule (I) selon la revendication 1 pour lesquels R₃ représente un atome d'hydrogène, leurs énantiomères, diastéréoisomères ainsi que leurs sels d'addition à un acide ou à une base pharmaceutiquement acceptable.

5. Composés de formule (I) selon la revendication 1 pour lesquels Rₐ représente un groupement hydroxy, leurs énantiomères, diastéréoisomères ainsi que leurs sels d'addition à un acide ou à une base pharmaceutiquement acceptable.

6. Composés de formule (I) selon la revendication 1 pour lesquels G₁ représente un groupement hétérocycloalkyle de formule 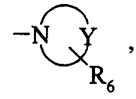 leur énantiomères, diastéréoisomères ainsi que leurs sels d'addition à un acide ou à une base pharmaceutiquement acceptable.

7. Composés de formule (I) selon la revendication 1 pour lesquels n et m valent chacun 2, Rₐ représente un groupement hydroxy, R₂ et R₃ représentent chacun un atome d'hydrogène, R₁ représente un atome d'halogène, et G₁ représente un groupement hétérocycloalkyle de formule 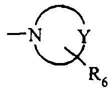 dans laquelle Y représente un atome d'azote ou un groupement -CH ou CH₂, et R₆ est choisi parmi les groupements alkyle, arylcarbonyle, arylcarbonylalkyle, diarylalkényle, (aryl)(hydroxy)alkyle, aryle, et hétéroaryle, leurs énantiomères, diastéréoisomères ainsi que leurs sels d'addition à un acide ou à une base pharmaceutiquement acceptable.

8. Composé de formule (I) selon la revendication 1 qui est l'acide 3-(6-{[(4-chlorophényl)sulfonyl]amino}-3-{2-[4-(6-fluoro-1,2-benzisothiazol-3-yl)-1-pipéridinyl]éthyl}-5,6,7,8-tétrahydro-1-naphtalényl)propanoïque.

9. Composé de formule (I) selon la revendication 1 qui est l'acide 3-(6-{[(4-chlorophényl)sulfonyl]amino}-3-{[2-(4-méthyl-1-pipérazinyl)phénoxy]méthyl}-5,6,7,8 -tétrahydro-1-naphtalényl)propanoïque.

10. Procédé de préparation des composés de formule (I) selon la revendication 1 **caractérisé en ce que** l'on utilise comme produit de départ un composé de formule (II) : 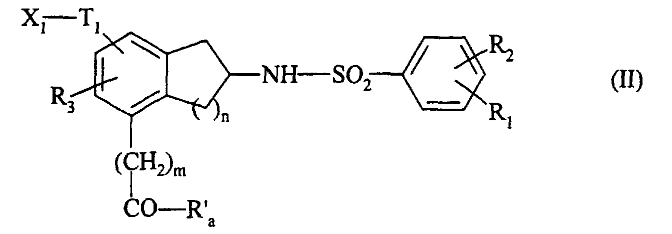 dans laquelle n, m, R₁, R₂, R₃ et T₁ sont tels que définis dans la formule (I), R'ₐ représente un groupement (C₁-C₆) alkoxy linéaire ou ramifié, et X₁ représente un groupe partant,
- qui, lorsque dans les composés de formule (I) que l'on souhaite obtenir G représente un groupement G₁ tel que défini dans la formule (I), est traité en milieu basique par un composé de formule G₁H, pour conduire au composé de formule (I/a): 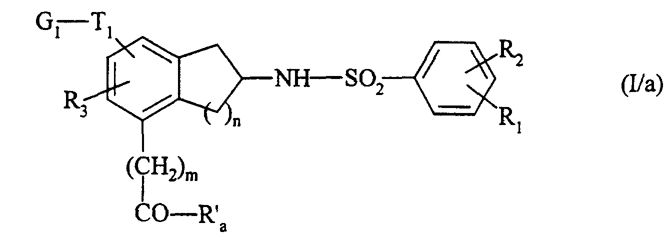 cas particulier des composés de formule (I) pour lequel m, n, R'ₐ, R₁, R₂, R₃, T₁, et G₁ sont tels que définis dans la formule (I),
- ou bien, lorsque dans les composés de formule (I) que l'on souhaite obtenir G représente un groupement G₁-T₂-A- tel que défini dans la formule (I), qui est traité en milieu basique par un composé de formule HO-T₂-A-G_{R}, dans laquelle T₂ et A sont tels que définis dans la formule (I) et G_{R} représente un groupement réactif choisi de façon à ce qu'il puisse réaliser la substitution nucléophile du groupe partant X₁ présent sur le substrat, pour conduire à un composé de formule (IV) : 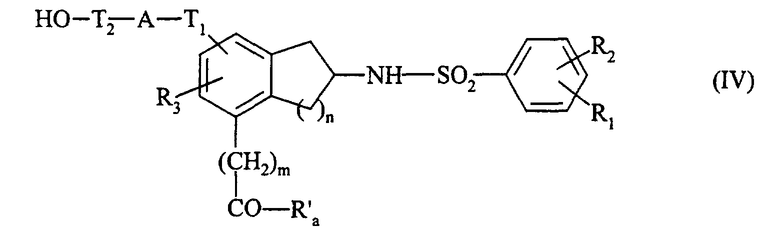
dans laquelle m, n, R'ₐ, R₁, R₂, R₃, T₁, A et T₂, sont tels que définis précédemment,
dont on transforme le groupement hydroxyle en groupe partant ou en atome d'halogène pour conduire au composé de formule (V) : 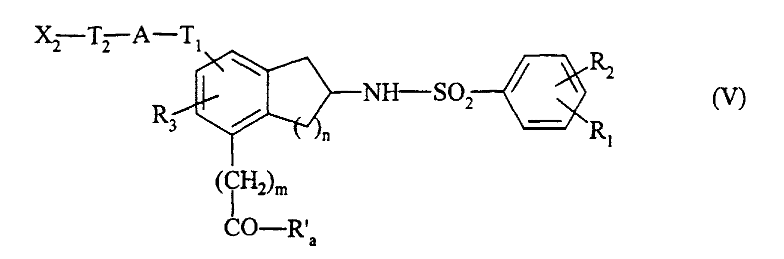 dans laquelle m, n, R'ₐ, R₁, R₂, R₃, T₁, A et T₂ sont tels que définis précédemment, et X₂ représente un groupe partant,
composé de formule (V) qui est traité en milieu basique par un composé de formule G₁H, G₁ étant tel que défini dans la formule (I), pour conduire à un composé de formule (I/b) : 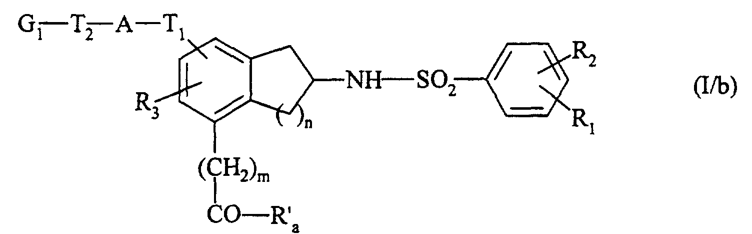 cas particulier des composés de formule (I) pour lequel m, n, R'ₐ, R₁, R₂, R₃, T₁, T₂, A, et G₁ sont tels que définis précédemment,
composés de formule (I/a) et (I/b), qui peuvent être soumis à une hydrolyse de la fonction ester, en milieu basique ou acide selon les groupements réactifs présents sur la molécule, pour conduire au composé de formule (I/c) : 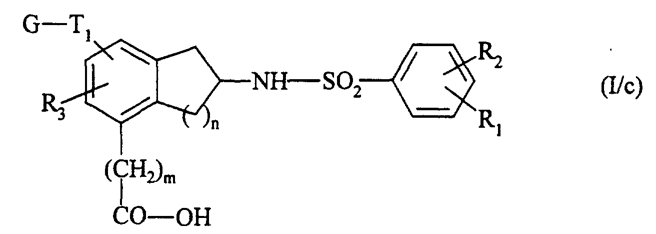 cas particulier des composés de formule (I) pour lequel m, n, R₁, R₂, R₃ et T₁ sont tels que définis précédemment, et G est tel que défini dans la formule (I),
composés (la), (I/b) et (I/c) formant la totalité des composés de formule (I), et :
- qui peuvent être, le cas échéant, purifiés selon une technique classique de purification,
- dont on sépare, le cas échéant, les stéréoisomères selon une technique classique de séparation,
- que l'on transforme, si on le souhaite, en leurs sels d'addition à un acide ou à une base pharmaceutiquement acceptable,
étant entendu qu'à tout moment jugé opportun au cours du procédé précédemment décrit, la fonction ester carboxylique -CO-R'ₐ peut être hydrolysée en acide correspondant, ce dernier pouvant être à nouveau transformé en un autre ester pour les besoins de la synthèse.

11. Compositions pharmaceutiques contenant comme principe actif un composé selon l'une quelconque des revendications 1 à 9 en combinaison avec un ou plusieurs excipients ou véhicules inertes, non toxiques, pharmaceutiquement acceptables.

12. Compositions pharmaceutiques selon la revendication 11 contenant un principe actif selon l'une quelconque des revendications 1 à 9 utiles pour la fabrication de médicaments utiles comme antagonistes des récepteurs du TXA₂ et des récepteurs 5-HT₂.

13. Compositions pharmaceutiques selon la revendication 11 contenant un principe actif selon l'une quelconque des revendications 1 à 9 utiles pour la fabrication de médicaments utiles dans le traitement des maladies cardiovasculaires athéro-thrombotiques telles que l'infarctus du myocarde, l'angine de poitrine, les accidents vasculaires cérébraux, la maladie de Raynaud, ou encore de l'asthme, des brochospasmes, de la migraine et des maladies veineuses.

## Patentansprüche

1. Verbindungen der Formel (I): 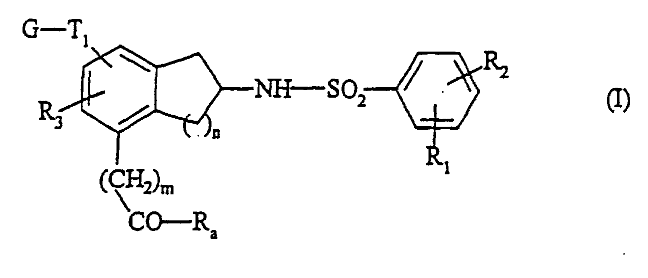 in der:
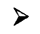 n eine ganze Zahl mit einem Wert zwischen 1 und 3 einschließlich darstellt,
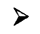 m eine ganze Zahl mit einem Wert zwischen 0 und 6 einschließlich bedeutet,
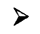 Rₐ eine Hydroxygruppe, geradkettige oder verzweigte (C₁-C₆)-Alkoxygruppe, Aryloxygruppe oder Arylalkyloxygruppe darstellt,
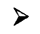 R₁ und R₂ unabhängig von einander ein Wasserstoffatom, ein Halogenatom, eine Alkylgruppe, geradkettige oder verzweigte (C₁-C₆)-Alkoxygruppe, Hydroxygruppe oder geradkettige oder verzweigte (C₁-C₆)-Perhalogenalkylgruppe bedeuten,
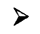 R₃ ein Wasserstoffatom oder eine Alkylgruppe, Arylalkylgruppe, Cycloalkylalkylgruppe, Arylgruppe oder Cycloalkylgruppe darstellt,
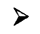 T₁ eine Alkylengruppe, O-Alkylengruppe, Alkylen-O-Gruppe oder (C₁-C₃)-Alkylen-O-(C₁-C₃)-alkylengruppe darstellt,
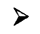 G eine Gruppe G₁- oder G₁-T₂-A- darstellt, worin:
* A eine Arylgruppe,
* T₂ eine Bindung oder eine Alkylengruppe, -O-alkylen-Gruppe, Alkylen-O-Gruppe oder (C₁-C₃)-Alkylen-O-(C₁-C₃)-alkylengruppe.
* G₁ eine Gruppe -NR₄R₅, worin R₄ und R₅ unabhängig von einander ein Wasserstoffatom, eine Alkylgruppe, Cycloalkylgruppe, gegebenenfalls substituierte Arylgruppe, gegebenenfalls substituierte Arylalkylgruppe, Cycloalkylalkylgruppe, gegebenenfalls substituierte Heteroarylgruppe oder gegebenenfalls substituierte Heteroarylalkylgruppe darstellen, oder G₁ eine Heterocycloalkylgruppe der Formel 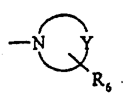 mit 5 bis 7 Kettengliedern, worin Y ein Stickstoffatom, Sauerstoffatom oder eine Gruppe CH oder CH₂ und R₆ ein Wasserstoffatom, eine Alkylgruppe, Cycloalkylgruppe, Cycloalkylalkylgruppe, gegebenenfalls substituierte Arylgruppe, gegebenenfalls substituierte Arylalkylgruppe, gegebenenfalls substituierte Arylcarbonylgruppe, gegebenenfalls substituierte Arylcarbonylalkylgruppe, gegebenenfalls substituierte Diarylalkylgruppe, gegebenenfalls substituierte Diarylalkenylgruppe, gegebenenfalls substituierte (Aryl)-(hydroxy)-alkyl-Gruppe, gegebenenfalls substituierte Heteroarylgruppe, gegebenenfalls substituierte Heteroarylalkylgruppe, gegebenenfalls substituierte Heteroarylcarbonylgruppe oder gegebenenfalls substituierte Heteroarylcarbonylalkylgruppe darstellen, bedeuten,
deren Enantiomere, Diastereoisomere sowie deren Additionssalze mit einer pharmazeutisch annehmbaren Säure oder Base,
mit der Maßgabe, daß:
- der Begriff Alkyl für eine geradkettige oder verzweigte Kette mit 1 bis 6 Kohlenstoffatomen steht,
- der Begriff Alkenyl für eine Kette mit 2 bis 6 Kohlenstoffatomen, die 1 bis 3 Doppelbindungen enthält, steht,
- der Begriff Alkylen für eine geradkettige oder verzweigte zweiwertige Gruppe steht, die 1 bis 6 Kohlenstoffatome aufweist, wenn nichts anderes angegeben ist,
- der Begriff Cycloalkyl für eine gesättigte cyclische Gruppe steht, die 3 bis 8 Kohlenstoffatome aufweist,
- der Begriff Aryl für eine Phenyl- oder Naphthyl-Gruppe steht,
- der Begriff Heteroaryl für eine mono- oder bicyclische ungesättigte oder teilweise gesättigte Gruppe mit 4 bis 11 Kettengliedern steht, die 1 bis 5 Heteroatome ausgewählt aus Stickstoff, Sauerstoff und Schwefel enthält,
- die Begriffe Diarylalkyl und Diarylalkenyl für Alkylgruppen beziehungsweise Alkenylgruppen, wie sie oben definiert worden sind, stehen, die durch zwei gleichartige oder verschiedene Arylgruppen, wie sie oben definiert worden sind, substituiert sind,
- der auf die Begriffe Aryl, Arylalkyl. Arylcarbonyl, Arylcarbonylalkyl, Diarylalkyl. Diarylalkenyl, Heteroaryl, Heteroarylalkyl, Heteroarylcarbonyl und Heterarylcarbonylalkyl bezogene Ausdruck "substituiert" bedeutet, daß die betreffenden Gruppen am aromatischen Teil durch eines oder mehrere Halogenatome, Alkylgruppen, geradkettige oder verzweigte (C₁-C₆)-Alkoxygruppen, Hydroxygruppen, Cyanogruppen, Nitrogruppen oder (gegebenenfalls durch ein oder zwei Alkylgruppen substituierte) Aminogruppen substituiert sind, wobei es sich versteht, daß die Heteroarylgruppen und Heteroarylalkylgruppen zusätzlich durch eine Oxogruppe substituiert sein können.

2. Verbindungen der Formel (I) nach Anspruch 1, worin n den Wert 2 besitzt, deren Enantiomere, Diastereoisomere sowie deren Additionssalze mit einer pharmazeutisch annehmbaren Säure oder Base.

3. Verbindungen der Formel (I) nach Anspruch 1, worin m den Wert 2 besitzt, deren Enantiomere, Diastereoisomere sowie deren Additionssalze mit einer pharmazeutisch annehmbaren Säure oder Base.

4. Verbindungen der Formel (I) nach Anspruch 1, worin R₃ ein Wasserstoffatom bedeutet, deren Enantiomere, Diastereoisomere sowie deren Additionssalze mit einer pharmazeutisch annehmbaren Säure oder Base.

5. Verbindungen der Formel (I) nach Anspruch 1, worin Rₐ eine Hydroxygruppe darstellt, deren Enantiomere, Diastereoisomere sowie deren Additionssalze mit einer pharmazeutisch annehmbaren Säure oder Base.

6. Verbindungen der Formel (I) nach Anspruch 1, worin G₁ eine Heterocycloalkylgruppe der Formel 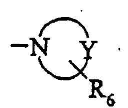 bedeutet, deren Enantiomere, Diastereoisomere sowie deren Additionssalze mit einer pharmazeutisch annehmbaren Säure oder Base.

7. Verbindungen der Formel (I) nach Anspruch 1, worin n und m jeweils den Wert 2 besitzen, Rₐ eine Hydroxygruppe bedeutet, R₂ und R₃ jeweils ein Wasserstoffatom darstellen; R₁ ein Halogenatom bedeutet und G₁ eine Heterocycloalkylgruppe der Formel 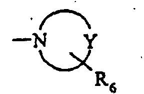 darstellt, wobei Y ein Stickstoffatom oder eine Gruppe -CH oder CH₂ darstellt und R₆ ausgewählt ist aus Alkylgruppen, Arylcarbonylgruppen, Arylcarbonylalkylgruppen, Diarylalkenylgruppen, (Aryl)-(hydroxy)-alkylgruppen, Arylgruppen und Heteroarylgruppen, deren Enantiomere, Diastereoisomere sowie deren Additionssalze mit einer pharmazeutisch annehmbaren Säure oder Base.

8. Verbindung der Formel (I) nach Anspruch 1, nämlich 3-(6-{[(4-Chlorphenyl)-sulfonyl]-amino}-3-{2-[4-(6-fluor-1,2-benzisothiazol-3-yl)-1-piperidinyl]-ethyl}-5,6,7,8-tetrahydro-1-naphthalinyl)-propansäure.

9. Verbindung der Formel (I) nach Anspruch 1, nämlich 3-(6-{[(4-Chlorphenyl)-sulfonyl]-amino}-3-{[2-[4-methyl-1-piperazinyl)-phenoxy]-methyl}-5,6,7,8-tetrahydro-1-naphthalinyl)-propansäure.

10. Verfahren zur Herstellung der Verbindungen der Formel (I) nach Anspruch 1, **dadurch gekennzeichnet, daß** man als Ausgangsprodukt eine Verbindung der Formel (II) verwendet: 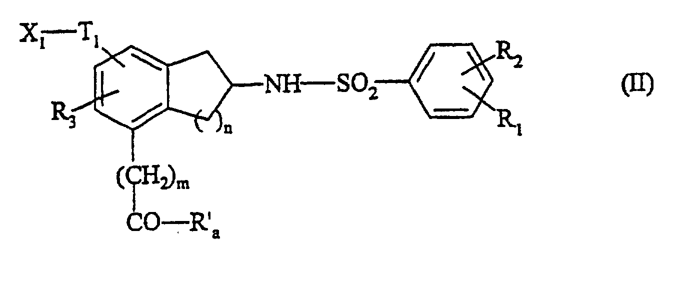 in der n, m, R₁, R₂, R₃ und T₁ die bezüglich der Formel (I) angegebenen Bedeutungen besitzen, R'ₐ eine geradkettige oder verzweigte (C₁-C₆)-Alkoxygruppe und X₁ eine austretende Gruppe bedeuten,
- welche man, wenn in den herzustellenden Verbindungen der Formel (I) G eine Gruppe G₁ darstellt, wie sie bezüglich der Formel (I) definiert worden ist, in basischem Medium mit einer Verbindung der Formel G₁H behandelt zur Bildung der Verbindung der Formel (I/a): 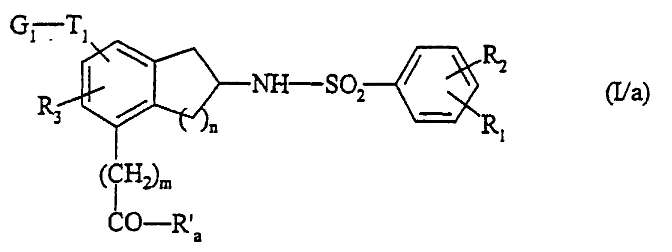 einem Sonderfall der Verbindungen der Formel (I), worin m, n, R'ₐ, R₁, R₂, R₃, T₁ und G₁ die bezüglich der Formel (I) angegebenen Bedeutungen besitzen,
- oder, wenn bei den herzustellenden Verbindungen der Formel (I) G eine Gruppe G₁-T₂-A- darstellt, wie bezüglich der Formel (I) definiert worden ist, man in basischem Medium mit einer Verbindung der Formel HO-T₂-A-G_{R} behandelt, in der T₂ und A die bezüglich der Formel (I) angegebenen Bedeutungen besitzen und G_{R} eine reaktive Gruppe darstellt, die in der Weise ausgewählt ist, daß sie eine nucleophile Substitution der an dem Substrat vorhandenen austretenden Gruppe X₁ ermöglicht, zur Bildung einer Verbindung der Formel (IV): 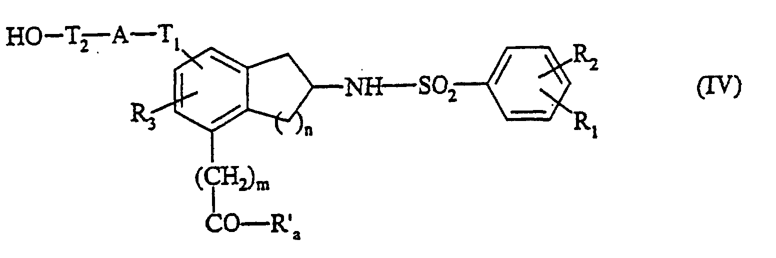 in der m, n, R'ₐ, R₁, R₂, R₃, T₁, A und T₂ die oben angegebenen Bedeutungen besitzen,
deren Hydroxylgruppe man in eine austretende Gruppe oder ein Halogenatom umwandelt, so daß man die Verbindung der Formel (V) erhält: 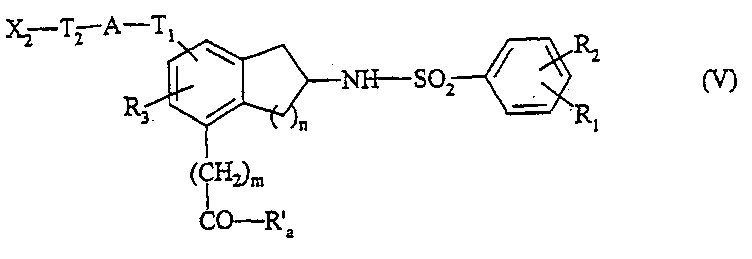 in der m, n, R'ₐ, R₁, R₂, K₃, T₁, A und T₂ die oben angegebenen Bedeutungen besitzen und X₂ eine austretende Gruppe darstellt,
welche Verbindung der Formel (V) in basischem Medium mit einer Verbindung der Formel G₁H behandelt wird, worin G₁ die bezüglich der Formel (I) angegebenen Bedeutungen besitzt, zur Bildung einer Verbindung der Formel (I/b): 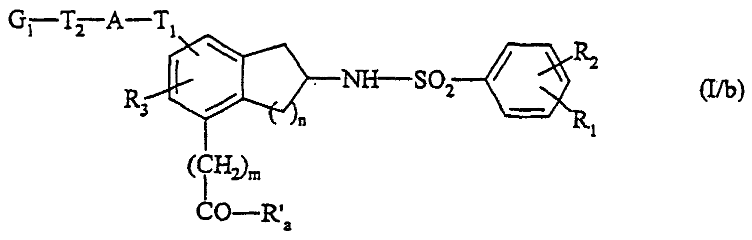 einem Sonderfall der Verbindungen der Formel (I), worin m, n. R'ₐ, R₁, R₂, R₃, T₁, T₂, A und G₁ die oben angegebenen Bedeutungen besitzen,
welche Verbindung der Formeln (I/a) und (I/b) einer Hydrolyse der Esterfunktion in basischem oder saurem Medium in Abhängigkeit von den an dem Molekül vorhandenen reaktiven Gruppen unterworfen werden können zur Bildung der Verbindung der Formel (I/c): 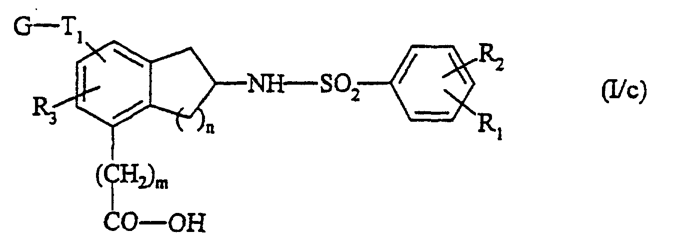 einem Sonderfall der Verbindungen der Formel (I), worin m, n, R₁, R₂, R₃, und T₁ die oben angegebenen Bedeutungen besitzen und G die bezüglich der Formel (I) angegebenen Bedeutungen aufweist,
welche Verbindungen (I/a), (I/b) und (I/c) die Gesamtheit der Verbindungen der Formel (I) bilden und welche:
- gegebenenfalls mit Hilfe einer klassischen Reinigungsmethode gereinigt weden können,
- man gegebenenfalls mit Hilfe einer klassischen Trennmethode in ihre Stereoisomeren auftrennen kann,
- man gewünschtenfalls in ihre Additionssalze mit einer pharmazeutisch annehmbaren Säure oder Base umwandeln kann,
wobei es sich versteht, daß man in jedem geeigneten Augenblick im Verlaufe des oben beschriebenen Verfahrens die Carbonsäureester-Funktion -CO-R'ₐ zu der entsprechenden Säure hydrolysieren kann, welche letztere erneut zum Zwecke der Synthese in einen anderen Ester umgewandelt werden kann.

11. Pharmazeutische Zubereitungen enthaltend als Wirkstoff eine Verbindung nach einem der Ansprüche 1 bis 9 in Kombination mit einem oder mehreren inerten, nichttoxischen, pharmazeutisch annehmbaren Trägermaterialien oder Bindemitteln.

12. Pharmazeutische Zubereitungen nach Anspruch 11, enthaltend einen Wirkstoff nach einem der Ansprüche 1 bis 9 für die Herstellung von Arzneimitteln, die als Antagonisten der Rezeptoren für TXA₂ und der Rezeptoren 5-HT₂ geeignet sind.

13. Pharmazeutische Zubereitungen nach Anspruch 11, enthaltend einen Wirkstoff nach einem der Ansprüche 1 bis 9 für die Herstellung von Arzneimitteln, die für die Behandlung von atherothrombotischen cardiovaskulären Erkrankungen. wie Myocardinfarkt, Angina pectoris, Cerebralgefäßvorfällen, der Raynaudschen Krankheit oder schließlich von Asthma, Bronchospasmen, der Migräne und Venenerkrankungen geeignet sind.

## Claims

1. Compounds of formula (I) : 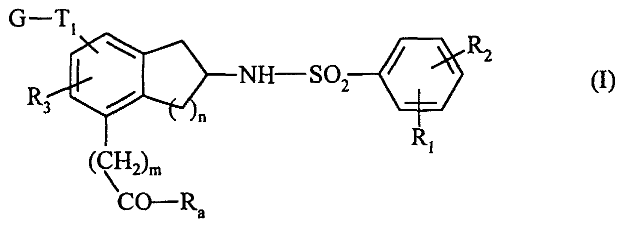 wherein:
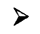 n is an integer of from 1 to 3 inclusive,
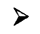 m is an integer of from 0 to 6 inclusive,
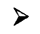 Rₐ represents a hydroxy, linear or branched (C₁-C₆)alkoxy, aryloxy or arylalkyloxy group,
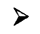 R₁ and R₂ represent independently a hydrogen atom, a halogen atom, an alkyl group, a linear or branched (C₁-C₆)alkoxy group, a hydroxy group or a linear or branched (C₁-C₆)perhaloalkyl group,
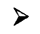 R₃ represents a hydrogen atom or an alkyl, arylalkyl, cycloalkylalkyl, aryl or cycloalkyl group,
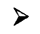 T₁ represents an alkylene, O-alkylene, alkylene-O- or (C₁-C₃)alkylene-O-(C₁-C₃)-alkylene group,
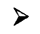 G represents a G₁- or G₁-T₂-A- group wherein :
* A represents an aryl group,
* T₂ represents a bond or an alkylene, -O-alkylene, alkylene-O- or (C₁-C₃)alkylene-O-(C₁-C₃)alkylene group,
* G₁ represents a -NR₄R₅ group wherein R₄ and R₅ represent independently a hydrogen atom, or an alkyl, cycloalkyl, optionally substituted aryl, optionally substituted arylalkyl, cycloalkylalkyl, optionally substituted heteroaryl or optionally substituted heteroarylalkyl group,
or G₁ represents a heterocycloalkyl group of formula 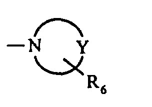 having from 5 to 7 ring members, wherein Y represents a nitrogen atom, an oxygen atom or a CH or CH₂ group and R₆ represents a hydrogen atom or an alkyl, cycloalkyl, cycloalkylalkyl, optionally substituted aryl, optionally substituted arylalkyl, optionally substituted arylcarbonyl, optionally substituted arylcarbonylalkyl, optionally substituted diarylalkyl, optionally substituted diarylalkenyl, optionally substituted (aryl)(hydroxy)alkyl, optionally substituted heteroaryl, optionally substituted heteroarylalkyl, optionally substituted heteroarylcarbonyl or optionally substituted heteroarylcarbonylalkyl group,
their enantiomers and diastereoisomers, and also addition salts thereof with a pharmaceutically acceptable acid or base,
wherein:
- the term "alkyl" denotes a linear or branched chain having from 1 to 6 carbon atoms,
- the term "alkenyl" denotes a chain having from 2 to 6 carbon atoms and containing from 1 to 3 double bonds,
- the term "alkylene" denotes a linear or branched divalent group containing from 1 to 6 carbon atoms, unless specified otherwise,
- the term "cycloalkyl" denotes a saturated cyclic group containing from 3 to 8 carbon atoms,
- the term "aryl" denotes a phenyl or naphthyl group,
- the term "heteroaryl" denotes a mono- or bi-cyclic group having from 4 to 11 ring members that is unsaturated or partially saturated and contains from 1 to 5 hetero atoms selected from nitrogen, oxygen and sulphur,
- the terms "diarylalkyl" and "diarylalkenyl" denote, respectively, alkyl and alkenyl groups as defined hereinbefore, substituted by two identical or different aryl groups as defined hereinbefore,
- the term "substituted" relating to aryl, arylalkyl, arylcarbonyl, arylcarbonylalkyl, diarylalkyl, diarylalkenyl, heteroaryl, heteroarylalkyl, heteroarylcarbonyl and heteroarylcarbonylalkyl denotes that the groups in question are substituted on the aromatic moiety by one or more halogen atoms, alkyl, linear or branched (C₁-C₆)alkoxy, hydroxy, cyano, nitro or amino group (amino optionally substituted by one or two alkyl groups), wherein the heteroaryl and heteroarylalkyl groups may in addition be substituted by an oxo group.

2. Compounds of formula (I) according to claim 1, wherein n is 2, their enantiomers and diastereoisomers, and also addition salts thereof with a pharmaceutically acceptable acid or base.

3. Compounds of formula (I) according to claim 1, wherein m is 2, their enantiomers and diastereoisomers, and also addition salts thereof with a pharmaceutically acceptable acid or base.

4. Compounds of formula (I) according to claim 1, wherein R₃ represents a hydrogen atom, their enantiomers and diastereoisomers, and also addition salts thereof with a pharmaceutically acceptable acid or base.

5. Compounds of formula (I) according to claim 1, wherein Rₐ represents a hydroxy group, their enantiomers and diastereoisomers, and also addition salts thereof with a pharmaceutically acceptable acid or base.

6. Compounds of formula (I) according to claim 1, wherein G₁ represents a heterocycloalkyl group of formula 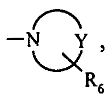 their enantiomers and diastereoisomers, and also addition salts thereof with a pharmaceutically acceptable acid or base.

7. Compounds of formula (I) according to claim 1, wherein n and m are each 2, Rₐ represents a hydroxy group, R₂ and R₃ each represents a hydrogen atom, R₁ represents a halogen atom and G₁ represents a heterocycloalkyl group of formula 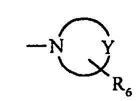 wherein Y represents a nitrogen atom or a -CH or CH₂ group and R₆ is selected from the groups alkyl, arylcarbonyl, arylcarbonylalkyl, diarylalkenyl, (aryl)-(hydroxy)alkyl, aryl and heteroaryl, their enantiomers and diastereoisomers, and also addition salts thereof with a pharmaceutically acceptable acid or base.

8. Compound of formula (I) according to claim 1, that is 3-(6-{[(4-chlorophenyl)-sulphonyl]amino}-3-{2-[4-(6-fluoro-1,2-benzisothiazol-3-yl)-1-piperidinyl]ethyl}-5,6,7,8-tetrahydro-1-naphthyl)propanoic acid.

9. Compound of formula (I) according to claim 1, that is 3-(6-{[(4-chlorophenyl)-sulphonyl]amino}-3-{[2-(4-methyl-1-piperazinyl)phenoxy]methyl}-5,6,7,8-tetrahydro-1-naphthyl)propanoic acid.

10. Process for the preparation of the compounds of formula (I) according to claim 1, **characterised in that** there is used as starting material a compound of formula (II): 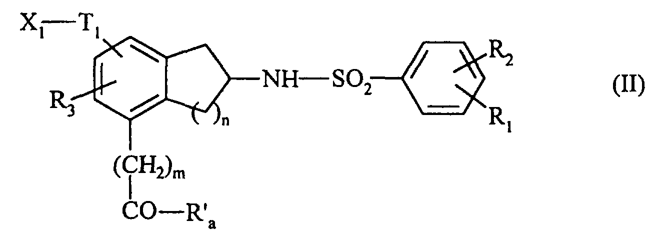 wherein n, m, R₁, R₂, R₃ and T₁ are as defined for formula (I), R'ₐ represents a linear or branched (C₁-C₆)alkoxy group and X₁ represents a leaving group,
- which, when it is desired to obtain compounds of formula (I) wherein G represents a group G₁ as defined for formula (I), is treated in basic medium with a compound of formula G₁H to yield a compound of formula (I/a): 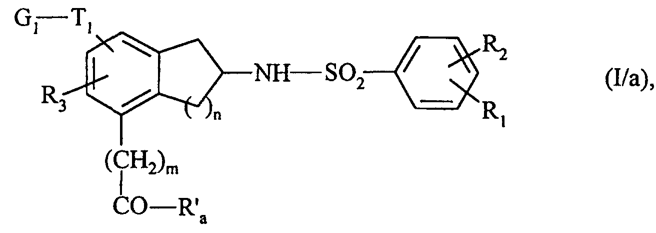 a particular case of the compounds of formula (I) wherein m, n, R'ₐ, R₁, R₂, R₃, T₁ and G₁ are as defined for formula (I),
- or which, when it is desired to obtain compounds of formula (I) wherein G represents a group G₁-T₂-A- as defined for formula (I), is treated in basic medium with a compound of formula HO-T₂-A-G_{R}, wherein T₂ and A are as defined for formula (I) and G_{R} represents a reactive group so selected that it can effect nucleophilic substitution of the leaving group X₁ present in the substrate to yield a compound of formula (IV) : 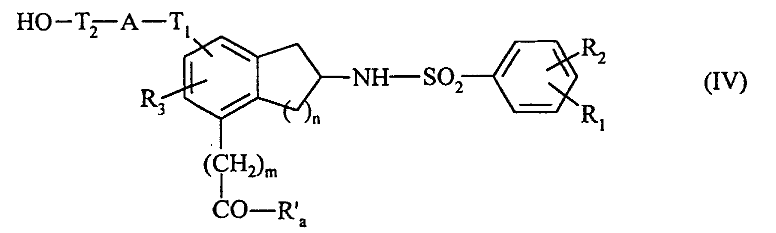
wherein m, n, R'ₐ, R₁, R₂, R₃, T₁, A and T₂ are as defined hereinbefore,
the hydroxy group of which is converted into a leaving group or into a halogen atom to yield a compound of formula (V) : 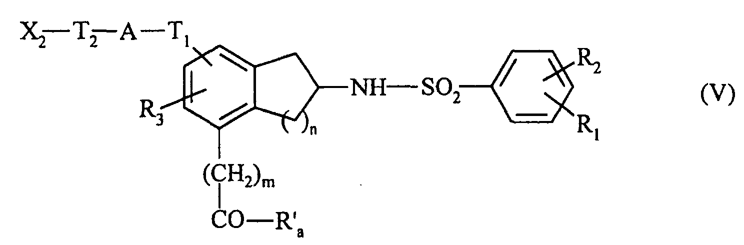 wherein m, n, R'ₐ, R₁, R₂, R₃, T₁, A and T₂ are as defined hereinbefore and X₂ represents a leaving group,
which compound of formula (V) is treated in basic medium with a compound of formula G₁H, G₁ being as defined for formula (I), to yield a compound of formula (I/b): 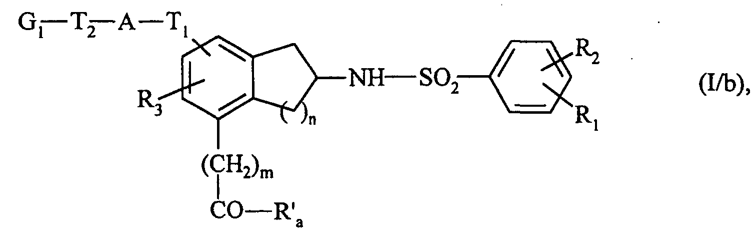 a particular case of the compounds of formula (I) wherein m, n, R'ₐ, R₁, R₂, R₃, T₁, T₂, A, and G₁ are as defined hereinbefore,
which compounds of formulae (I/a) and (I/b) may be subjected to hydrolysis of the ester function, in acid or basic medium according to the reactive groups present in the molecule, to yield a compound of formula (I/c): 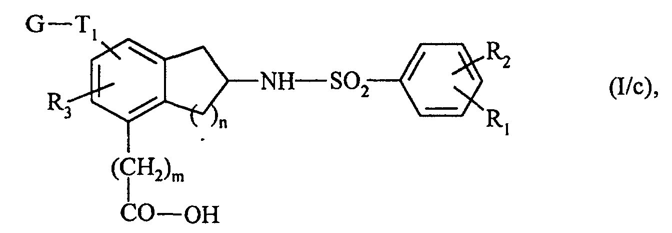 a particular case of the compounds of formula (I) wherein m, n, R₁, R₂, R₃ and T₁ are as defined hereinbefore and G is as defined for formula (I),
which compounds (Ia), (I/b) and (I/c) constitute the totality of the compounds of formula (I), and :
- may, if necessary, be purified according to a conventional purification technique,
- are optionally separated into their stereoisomers according to a conventional separation technique,
- are converted, if desired, into addition salts with a pharmaceutically acceptable acid or base,
wherein, at any moment considered appropriate during the course of the process described above, the carboxylic ester function -CO-R'ₐ may be hydrolysed to the corresponding acid, which may be converted again to a different ester as required by the synthesis.

11. Pharmaceutical compositions comprising as active ingredient a compound according to any one of claims 1 to 9 in combination with one or more pharmaceutically acceptable, inert, non-toxic excipients or carriers.

12. Pharmaceutical compositions according to claim 11, comprising an active ingredient according to any one of claims 1 to 9 for use in the manufacture of medicaments useful as TXA₂ receptor and 5-HT₂ receptor antagonists.

13. Pharmaceutical compositions according to claim 11, comprising an active ingredient according to any one of claims 1 to 9 for use in the manufacture of medicaments useful in the treatment of atherothrombotic cardiovascular disorders, such as myocardial infarction, angina pectoris, cerebral vascular accidents, Raynaud's disease, and also asthma, bronchospasms, migraine and venous disorders.
